# EUROPÄISCHE PATENTSCHRIFT

(11) **EP 2 732 811 B1**
(45) Veröffentlichungstag und Bekanntmachung des Hinweises auf die Patenterteilung: **13.01.2016**
(21) Anmeldenummer: 12193209.9
(22) Anmeldetag: 19.11.2012
(51) Int. Cl.: A61K 9/19, A61K 9/00, A61K 31/706

(54) **Verfahren zur Herstellung einer gefriergetrockneten Zusammensetzung**
Method for producing a freeze-dried compound
Procédé de fabrication d'une composition lyophilisée

(43) Veröffentlichungstag der Anmeldung: 21.05.2014
(73) Patentinhaber: Oncotec Pharma Produktion GmbH, 06861 Dessau-Roßlau (DE)
(72) Erfinder: Schmidt, Sven, 04229 Leipzig (DE); Richter, Christin, 06785 Oranienbaum-Wörlitz (DE); Beck, Matthias, 04509 Zwochau (DE); Gewert, Jan-Arne, 79576 Weil am Rhein (DE); Hans, Jörg, 25462 Rellingen (DE)
(74) Vertreter: UEXKÜLL & STOLBERG

(56) Entgegenhaltungen:
- WO-A1-2010/036702
- WO-A2-2006/076620
- US-A- 4 537 883
- MAAS B: "Stabilität von Bendamustinhydrochlorid in Infusionsl sungen", DIE PHARMAZIE, GOVI VERLAG PHARMAZEUTISCHER VERLAG GMBH, ESCHBORN, DE, Bd. 49, Nr. 10, 1. Januar 1994 (1994-01-01), Seiten 775-777, XP008060268, ISSN: 0031-7144

## Beschreibung

Die Erfindung betrifft ein Verfahren zur Herstellung einer gefriergetrockneten Zusammensetzung mit Gehalt an Bendamustin oder einem Salz davon, welches insbesondere in kurzer Zeit zu leicht und schnell rekonstituierbaren Produkten führt.

Bei Bendamustin handelt es sich um ein antitumorales Chemotherapeutikum aus der Gruppe der Alkylantien. Es zeichnet sich vor allem durch ein sehr gutes Nebenwirkungsprofil aus. Es wird in Form von wäßrigen Injektions- oder Infusionslösungen eingesetzt. Allerdings sind derartige wäßrige Lösungen nicht lange haltbar, weil Bendamustin und seine Salze hydrolyseempfindlich sind. Aus diesem Grund wird Bendamustin regelmäßig in gefriergetrockneter Form hergestellt und dieses Lyophilisat wird unmittelbar vor der Verabreichung mit sterilem Lösungsmittel rekonstituiert, um eine Injektions- oder Infusionslösung zu ergeben.

Die bekannten Verfahren zur Herstellung von gefriergetrockneten Bendamustin-Zusammensetzungen und die damit erhaltenen Produkte haben jedoch eine Reihe von Nachteilen.

Die WO 2006/076620 beschreibt Lyophilisate von Bendamustin und Gefriertrocknungsverfahren zu deren Herstellung. Die beschriebenen Lyophilisate benötigen für die vollständige Rekonstitution allerdings einen Zeitraum von 3-5 Minuten. Außerdem wird bei Untersuchungen von verschiedenen Verfahren das Problem von Pulverauswurf während der Haupttrocknung festgestellt. Diesem Problem wird durch Verwendung einer stufenweisen und langsamen Haupttrocknung begegnet. Es wird demgemäß ein Gefriertrocknungsverfahren beschrieben, welches sich einer Haupttrocknung in mehreren Schritten bei Temperaturen von -20°C, -15°C und - 12°C bedient und insgesamt etwa 89 Stunden dauert. Eine derart lange Verfahrensdauer ist jedoch insbesondere aus wirtschaftlichen Gründen sehr nachteilig.

Weiter ist auch aus der WO 2011/103150 ein Verfahren zur Herstellung von Bendamustin-Lyophilisaten bekannt. Im Rahmen des Verfahrens erfolgt eine Trocknung in zwei Stufen bei 25°C und 30°C, wobei die gesamte Verfahrensdauer mehr als 60 Stunden beträgt. Weiter ist in den Lyophilisaten allerdings zwingend ein Gehalt an Cyclodextrin erforderlich, um die gewünschte Stabilität und eine schnelle Rekonstitution zu Injektionslösungen zu gewährleisten.

Die aus dem Stand der Technik bekannten Verfahren erweisen sich demgemäß als langwierig. Lange Prozesszeiten sind aber gerade bei Fertigung im industriellen Maßstab sehr ungünstig. Die mit den bekannten Verfahren erhaltenen Lyophilisate benötigen zudem sehr lange zur vollständigen Rekonstitution oder bedürfen besonderer Hilfsstoffe wie Cyclodextrin, um eine schnelle Auflösung sicherzustellen.

Erfindungsgemäß sollen diese Probleme vermieden werden. Der Erfindung liegt insbesondere die Aufgabe zugrunde, ein Verfahren zur Herstellung gefriergetrockneter Zusammensetzungen mit Gehalt an Bendamustin oder einem Salz davon zur Verfügung zu stellen, welches sich durch eine verkürzte Dauer auszeichnet und welches zu Zusammensetzungen führt, die innerhalb kurzer Zeit zu Lösungen rekonstituiert werden können.

Diese Aufgabe wird durch das Verfahren nach den Ansprüchen 1 bis 15 gelöst. Gegenstand der Erfindung ist ebenfalls die gefriergetrocknete Zusammensetzung nach Anspruch 16.

Das erfindungsgemäße Verfahren zur Herstellung einer gefriergetrockneten Zusammensetzung mit Gehalt an Bendamustin oder einem Salz davon ist dadurch gekennzeichnet, dass
(a) eine Lösung mit Gehalt an Bendamustin oder einem Salz davon bei einer ersten Gefriertemperatur gefroren wird,
(b) optional
   (b1) die gefrorene Lösung bei einer Temperatur von -25 bis -10°C getempert und
   (b2) bei einer zweiten Gefriertemperatur gefroren wird, die niedriger als die Temperatur in (b1) ist, und
   (c) die nach Stufe (a) oder (b) erhaltene gefrorene Lösung bei einer ersten Trocknungstemperatur von -5 bis +5°C für einen Zeitraum von mindestens 10 Stunden getrocknet wird, um eine gefriergetrocknete Zusammensetzung zu erzeugen.

In der Stufe (a) wird eine Lösung eingesetzt, die Bendamustin oder ein Salz davon enthält. Bevorzugt wird Bendamustin Hydrochlorid verwendet. Das Bendamustin oder das Salz davon liegt insbesondere in einer Konzentration von 5,65 bis 5,70 mg/ml und bevorzugt 5,67 bis 5,69 mg/ml in der Lösung vor.

Die Lösung enthält vorzugsweise mindestens ein organisches Lösungsmittel. Das organische Lösungsmittel ist insbesondere ein Alkohol, vorzugsweise Ethanol, Propanol oder tert.-Butanol und besonders bevorzugt Ethanol. Der Gehalt an Alkohol in der Lösung beträgt vorzugsweise 1,5 bis 2,5 Vol.-% und besonders bevorzugt 1,8 bis 2,0 Vol.-%.

Die Lösung enthält üblicherweise auch Wasser. Der Gehalt an Wasser in der Lösung beträgt insbesondere 96,5 bis 97,5 Vol.-% und besonders bevorzugt 97,0 bis 97,2 Vol.-%.

Weiter ist es bevorzugt, dass die Lösung mindestes ein Monosaccharid, Oligosaccarid oder Zuckeralkohol, vorzugsweise Sucrose, Dextrose, Maltose, Lactose, Sorbit, Mannit oder Dextran, und besonders bevorzugt Mannit enthält. Diese Substanzen können als Gerüstbildner bezeichnet werden, und sie können den Gefriertrocknungsprozess hinsichtlich der mechanischen Stabilität der gefriergetrockneten Zusammensetzung günstig beeinflussen. Es wird angenommen, dass dieser positive Einfluss über die Bildung von teilamorphen Strukturen erfolgt. Die Gerüstbildner sind insbesondere in einer Konzentration von 6,75 bis 6,90 mg/ml und bevorzugt 6,80 bis 6,84 mg/ml in der Lösung vorhanden.

In der Regel wird die Lösung in ein geeignetes Gefäß, z.B. eine Injektionsflasche, abgefüllt, und das Gefäß wird in einen üblichen Gefriertrockner mit kühl- und heizbaren Stellflächen gestellt, auf denen die Lösung den verschiedenen Temperaturen des Gefriertrocknungsverfahrens ausgesetzt werden kann.

Erfindungsgemäß wird die Lösung dann bei einer ersten Gefriertemperatur gefroren. Es ist bevorzugt, dass das Abkühlen auf die erste Gefriertemperatur mit einer Kühlrate von 0,6 bis 1,2 °C/min, bevorzugt 0,8 bis 1,0 °C/min und besonders bevorzugt etwa 0,9 °C/min erfolgt.

Es ist darüber hinaus bevorzugt, dass die erste Gefriertemperatur -40°C oder niedriger, vorzugsweise -40°C bis -55°C und besonders bevorzugt -40°C bis -50°C beträgt.

Es hat sich weiter als vorteilhaft erwiesen, die Lösung für einen Zeitraum von 90 bis 150 min, insbesondere 100 bis 120 min bei der ersten Gefriertemperatur zu frieren.

Die folgende Stufe (b) ist optional. Allerdings ist es bevorzugt, sie durchzuführen, da sie zur Erzielung von kurzen Rekonstitutionszeiten des schließlich erhaltenen Lyophilisats beiträgt. Es wird angenommen, dass dies über eine Aktivierung von Diffusions- und Kristallisationsvorgängen erfolgt, die zu einer günstigen Beeinflussung der Porengröße des Lyophilisats führen.

In der Stufe (b) wird die gefrorene Lösung (b1) bei einer Temperatur von -25 bis -10°C getempert und (b2) bei einer zweiten Gefriertemperatur erneut gefroren, die niedriger als die Temperatur in (b1) ist.

Die in Stufe (b1) eingesetzte Temperatur zum Tempern beträgt insbesondere -20 bis -13°C und besonders bevorzugt etwa -15°C. Es ist bevorzugt, die gefrorene Lösung für einen Zeitraum von mindestens 3 Stunden, vorzugsweise mindestens 4 Stunden und besonders bevorzugt mindestens 5 Stunden zu tempern.

Als zweite Gefriertemperatur in Stufe (b2) wird insbesondere eine Temperatur von -40°C oder niedriger, vorzugsweise -40°C bis - 55°C und besonders bevorzugt -40°C bis -50°C gewählt.

Es ist bevorzugt, die gefrorene Lösung für einen Zeitraum von mindestens 2 Stunden und vorzugsweise mindestens 3 Stunden der zweiten Gefriertemperatur auszusetzen.

Das Erwärmen der gefrorenen Lösung auf die in (b1) gewählte Temperatur für das Tempern erfolgt vorzugsweise mit einer Heizrate von 0,9 bis 1,5 °C/min, bevorzugt 1,1 bis 1,3 °C/min und besonders bevorzugt etwa 1,2 °C/min.

Das anschließende erneute Abkühlen auf die in (b2) gewählte zweite Gefriertemperatur erfolgt vorzugsweise mit einer Kühlrate von 0,9 bis 1,5 °C/min, bevorzugt 1,1 bis 1,3 °C/min und besonders bevorzugt etwa 1,2 °C/min.

Schließlich wird in Stufe (c) die nach Stufe (a) oder (b) erhaltene gefrorene Lösung bei einer ersten Trocknungstemperatur von -5 bis +5°C für einen Zeitraum von mindestens 10 Stunden getrocknet, um eine gefriergetrocknete Zusammensetzung zu erzeugen.

Es hat sich überraschenderweise herausgestellt, dass dieser spezielle Temperaturbereich offenbar wesentlich für die erzielte kurze Verfahrensdauer und die sehr günstigen Eigenschaften der erhaltenen gefriergetrockneten Zusammensetzung, d.h. des Lyophilisats, ist.

Die erste Trocknungstemperatur beträgt vorzugsweise -2 bis +2°C und besonders bevorzugt etwa 0°C.

Weiter hat es sich als vorteilhaft erwiesen, für einen Zeitraum von mindestens 12, insbesondere mindestens 14, vorzugsweise mindestens 16 und besonders bevorzugt mindestens 18 Stunden bei der ersten Trocknungstemperatur zu trocknen. Es ist weiter bevorzugt, für einen Zeitraum von bis zu 45 Stunden zu trocknen.

Die Erwärmung der gefrorenen Lösung auf die erste Trocknungstemperatur erfolgt insbesondere mit einer Heizrate von 0,38 bis 0,46°C/min, vorzugsweise 0,40 bis 0,44°C/min und besonders bevorzugt etwa 0,42°C/min.

Zur Herbeiführung der Trocknung wird üblicherweise die gefrorene Lösung in Stufe (c) einem verminderten Atmosphärendruck ausgesetzt. Dadurch kommt es in hohem Maße zur Sublimation von Wasser aus der Lösung, welches sich z.B. an dafür vorgesehenen kühleren Bereichen des Gefriertrockners niederschlägt.

Erfindungsgemäß ist es bevorzugt, während des Schrittes (c) die gefrorene Lösung einem verminderten Druck von 0,2 bis 0,3 mbar und insbesondere 0,26 bis 0,29 mbar auszusetzen.

In einer bevorzugten Ausführungsform des erfindungsgemäßen Verfahrens wird die nach Stufe (c) erhaltene gefriergetrocknete Zusammensetzung in der Stufe (d) bei einer zweiten Trocknungstemperatur von +20°C bis +40°C, vorzugsweise +30°C bis +40°C und besonders bevorzugt bei etwa +40°C weiter getrocknet.

Diese Nachtrocknung dient insbesondere der Entfernung von stärker gebundenem Wasser aus dem erhaltenen Lyophilisat.

Es ist bevorzugt, für einen Zeitraum von mindestens 6, vorzugsweise mindestens 8 und besonders bevorzugt mindestens 12 Stunden bei der zweiten Trocknungstemperatur zu trocknen. Weiter ist es bevorzugt, für einen Zeitraum von bis zu 15 Stunden zu trocknen.

Die Erwärmung der gefriergetrockneten Zusammensetzung auf die zweite Trocknungstemperatur erfolgt insbesondere mit einer Heizrate von 0,10 bis 0,25 °C/min, vorzugsweise 0,15 bis 0,20°C/min und besonders bevorzugt etwa 0,17°C/min.

Auch während der Trocknung bei der zweiten Trocknungstemperatur wird die erhaltene Zusammensetzung üblicherweise vermindertem Atmosphärendruck ausgesetzt. Damit wird die Effektivität der Trocknung gesteigert. Der Druck beträgt üblicherweise 0,03 bis 0,09 mbar und insbesondere 0,04 bis 0,06 mbar.

Nach Abschluss des erfindungsgemäßen Verfahrens lässt man in der Regel die gefriergetrocknete Zusammensetzung auf Raumtemperatur kommen und verschließt die sie enthaltende Behälter, wie z.B. Injektionsflaschen, unter sterilen Bedingungen.

Das erfindungsgemäße Verfahren kann in Gefriertrocknern durchgeführt werden, wie sie zur Herstellung von Lyophilisaten für pharmazeutische Zwecke verwendet werden. Sie verfügen über heizbare und kühlbare Stellflächen, auf die ein geeignetes Gefäß mit der zu trocknenden Lösung gestellt wird. Geeignete Gefriertrockner sind z.B. Gefriertrocknungsanlagen der Marke Klee (Optima Group pharma GmbH, Gladenbach) mit einer Stellflächenkapazität von 0,5 bis 1 m² und 14 bis 15 m².

Es hat sich überraschenderweise gezeigt, dass das erfindungsgemäße Verfahren sich von konventionellen Verfahren durch eine deutlich verkürzte Verfahrensdauer auszeichnet. Das stellt bei der industriellen Fertigung einen ganz wesentlichen Vorteil dar.

Das erfindungsgemäße Verfahren kann z.B. innerhalb eines Zeitraums von etwa 41 Stunden abgeschlossen werden, während die Verfahrensdauer konventioneller Verfahren bei mehr als 60 Stunden liegt.

Trotz der kurzen Verfahrensdauer hat die erfindungsgemäß erhaltene gefriergetrocknete Zusammensetzung einen nur geringen Gehalt an Restfeuchte. Es kann mit dem erfindungsgemäßen Verfahren eine Restfeuchte von nur etwa 0,25 % erzielt werden. Das ist von besonderem Vorteil für die Stabilität der Zusammensetzung bei Lagerung, da ein hoher Restgehalt an Wasser die Hydrolyse von Bendamustin oder eines Salzes davon und damit die Reduzierung des Wirkstoffgehalts sowie die Bildung unerwünschter Abbauprodukte begünstigen würde.

Die Restfeuchte der erfindungsgemäß erhaltenen Zusammensetzung beträgt insbesondere weniger als 1,0 %, bevorzugt weniger als 0,7 % und besonders bevorzugt weniger als 0,3 %.

Auch wurde überraschenderweise festgestellt, dass bei dem erfindungsgemäßen Verfahren die erzielte Restfeuchte innerhalb einer Charge kaum schwankt. Demgegenüber kommt es bei konventionellen Verfahren häufig zu deutlichen Schwankungen der Restfeuchte, was bei einem inakzeptabel hohen Wert sogar eine weitere Trocknung entsprechender Produkte erforderlich machen kann.

Die erfindungsgemäß erhaltene Zusammensetzung verfügt offenbar auch über eine besondere und vorteilhafte Struktur, da für deren Rekonstitution zu einer Lösung eine nur sehr kurzer Zeitspanne von insbesondere weniger als 20 Sekunden ausreicht. Das stellt einen weiteren wichtigen Vorteil dar, weil es damit Klinikpersonal möglich ist, Injektionslösungen unmittelbar vor der beabsichtigten Verabreichung an den Patienten frisch herzustellen, ohne lange Wartezeiten für eine vollständige Auflösung in Kauf nehmen zu müssen. Gleichfalls nimmt bei solchen kurzen Rekonstitutionszeiten das Risiko ab, dass es zu unerwünschten Abbaureaktion des Wirkstoffs kommt.

Für die Rekonstitution der Zusammensetzung wird üblicherweise Wasser für Injektionszwecke verwendet.

Aufgrund der geschilderten vorteilhaften Eigenschaften ist die Erfindung auch auf eine gefriergetrocknete Zusammensetzung mit Gehalt an Bendamustin oder einem Salz davon gerichtet, wobei die Zusammensetzung durch das erfindungsgemäße Verfahren erhältlich ist.

Diese Zusammensetzung liegt insbesondere in Form eines sie enthaltenden Behälters, vorzugsweise einer Injektionsflasche, vor.

Die Erfindung wird im Folgenden anhand von Beispielen näher erläutert.

### Beispiele

### Beispiel 1 - Zusammensetzung mit 25 mg Bendamustin

### (a) Herstellung der Lösung

Es wurde zunächst eine Lösung hergestellt, indem 2,84 g Bendamustin Hydrochlorid in einen Behälter eingewogen und nach Zugabe von 7,6 g Ethanol unter Rühren aufgelöst wurden. In einem separaten Behälter wurden 485,9 g Wasser für Injektionszwecke eingewogen und 3,41 g Mannit darin unter Rühren aufgelöst. Das Bendamustin-Ethanol Gemisch wurde in die Mannit-Lösung überführt und so lange gerührt, bis alle Bestandteile gelöst waren.

4,5 ml dieser Lösung wurden dann in eine Injektionsflasche mit einem Volumen von 20 ml abgefüllt.

Pro Injektionsflasche hatte die Lösung folgende Zusammensetzung:
25,56 mg Bendamustin Hydrochlorid
30,69 mg Mannit
68,4 mg Ethanol
4,373 g Wasser für Injektionszwecke

### (b) Gefriertrocknung

Dann wurde die Lösung gefriergetrocknet, indem nacheinander die folgenden Schritte in einem Gefriertrockner der Marke Klee mit einer Stellflächenkapazität von 0,6 m² durchgeführt wurden:
1. Aufstellen der befüllten Injektionsflasche auf die auf +3°C vorgekühlte Stellfläche des Gefriertrockners
2. Abkühlen auf -50°C mit einer Kühlrate von 0,9°C/min
3. Halten bei -50°C für 1 Stunde
4. Erwärmen auf -15°C mit einer Heizrate von 1,2°C/min
5. Halten bei -15°C für 3 Stunden
6. Abkühlen auf -50°C mit einer Abkühlrate von 1,2°C/min
7. Halten bei -50°C für 2 Stunden
8. Verringern des Atmosphärendruck auf 0,28 mbar
9. Erwärmen auf 0°C mit einer Heizrate von 0,42°C/min
10. Halten bei 0°C für 18 Stunden
11. Verringern des Atmosphärendrucks auf 0,05 mbar
12. Erwärmen auf +40°C mit einer Heizrate von 0,17°C/min
13. Halten bei +40°C für 8 Stunden
14. Belüften der Trocknungskammer des Gefriertrockners und Abkühlen auf Raumtemperatur
15. Verschließen der Injektionsflasche bei geringem Vakuum von 950 mbar

Die oben angegebenen Temperaturen, den die Lösung während des Gefriertrocknungszyklus ausgesetzt wird, beziehen sich auf die jeweilige Temperatur der Stellfläche.

Das gesamte Verfahren dauerte damit lediglich etwa 41 Stunden. Es wurde ein weißes pulvriges Lyophilisat erhalten. Dessen wesentliche Eigenschaften sind nachstehend aufgeführt.

### (c) Wesentliche Eigenschaften

Es wurde zunächst die Zeit ermittelt, die benötigt wird, um das erhaltene Lyophilisat zu rekonstituieren (Rekonstitutionszeit). Dazu wurden dem Lyophilisat 10 ml Wasser für Injektionszwecke zugegeben. Das Gemisch wurde geschüttelt, bis eine klare Lösung vorlag. Die dafür benötigte Zeit wurde ermittelt.

Weiter wurde der verbliebene Gehalt an Wasser (Restfeuchte) bestimmt. Dazu wurden zunächst 5 ml getrocknetes Methanol zum Lyophilisat zugegeben. Die erhaltene Suspension wurde in einen Titrator von Mettler Toledo überführt und über eine Karl-Fischer-Titration wurde die Restfeuchte in % bestimmt.

Die ermittelten Eigenschaften des Lyophilisats sowie die zu seiner Herstellung erforderliche Verfahrensdauer der Gefriertrockung waren wie folgt:

| **Parameter** | **Ergebnis** |
|---|---|
| Rekonstitutionszeit | 18 Sekunden |
| Restfeuchte | 0,66 % |
| Verfahrensdauer | etwa 41 Stunden |

Diese Eigenschaften zeigen, dass es erfindungsgemäß möglich ist, trotz der gegenüber konventionellen Verfahren verkürzten Verfahrensdauer eine gefriergetrocknete Zusammensetzung zu erzeugen, die sich in weniger als 20 Sekunden rekonstituieren lässt und zudem über eine nur geringe Restfeuchte verfügt. Damit kann aus der erfindungsgemäßen Zusammensetzung schnell eine Injektions- oder Infusionslösung erzeugt werden, und die geringe Restfeuchte fördert die Stabilität der erfindungsgemäßen Zusammensetzung gegenüber hydrolytischen Abbauprozessen.

### Beispiel 2 - Zusammensetzung mit 100 mg Bendamustin

### (a) Herstellung der Lösung

Es wurden 17,9 ml der gemäß Beispiel 1 hergestellten Lösung in eine Injektionsflasche mit einem Volumen von 50 ml abgefüllt. Pro Injektionsflasche hatte die Lösung folgende Zusammensetzung:
101,67 mg Bendamustin Hydrochlorid
122,08 mg Mannit
272,08 mg Ethanol
17,395 g Wasser für Injektionszwecke

### (b) Gefriertrocknung

Dann wurde die Lösung gefriergetrocknet, indem nacheinander die folgenden Schritte in einem Gefriertrockner der Marke Klee mit einer Stellflächenkapazität von 0,6 m² durchgeführt wurden:
1. Aufstellen der befüllten Injektionsflasche auf die auf +3°C vorgekühlte Stellfläche des Gefriertrockners
2. Abkühlen auf -50°C mit einer Kühlrate von 0,9°C/min
3. Halten bei -50°C für 1 Stunde
4. Erwärmen auf -15°C mit einer Heizrate von 1,2°C/min
5. Halten bei -15°C für 3 Stunden
6. Abkühlen auf -50°C mit einer Abkühlrate von 1,2°C/min
7. Halten bei -50°C für 3 Stunden
8. Verringern des Atmosphärendruck auf 0,28 mbar
9. Erwärmen auf 0°C mit einer Heizrate von 0,42°C/min
10. Halten bei 0°C für 32 Stunden
11. Verringern des Atmosphärendrucks auf 0,05 mbar
12. Erwärmen auf +40°C mit einer Heizrate von 0,17°C/min
13. Halten bei +40°C für 4 Stunden
14. Belüften der Trocknungskammer des Gefriertrockners und Abkühlen auf Raumtemperatur
15. Verschließen der Injektionsflasche bei geringem Vakuum von 950 mbar

Das gesamte Verfahren dauerte damit lediglich etwa 52 Stunden. Es wurde ein weißes pulvriges Lyophilisat erhalten. Dessen wesentliche Eigenschaften sind nachstehend aufgeführt.

### (c) Wesentliche Eigenschaften

Es wurde zunächst die Zeit ermittelt, die benötigt wird, um das erhaltene Lyophilisat zu rekonstituieren (Rekonstitutionszeit). Dazu wurde analog Beispiel 1 verfahren. Weiter wurde analog Beispiel 1 der verbliebene Gehalt an Wasser (Restfeuchte) bestimmt.

Die ermittelten Eigenschaften des Lyophilisats sowie die zu seiner Herstellung erforderliche Verfahrensdauer der Gefriertrocknung waren wie folgt:

| **Parameter** | **Ergebnis** |
|---|---|
| Rekonstitutionszeit | 17 Sekunden |
| Restfeuchte | 0,25 % |
| Verfahrensdauer | etwa 52 Stunden |

Diese Eigenschaften zeigen, dass auch diese erfindungsgemäße Zusammensetzung trotz geringer Verfahrensdauer innerhalb weniger als 20 Sekunden rekonstituierbar war und über eine nur geringe Restfeuchte verfügte.

## Patentansprüche

1. Verfahren zur Herstellung einer gefriergetrockneten Zusammensetzung mit Gehalt an Bendamustin oder einem Salz davon, bei dem
(a) eine Lösung mit Gehalt an Bendamustin oder einem Salz davon bei einer ersten Gefriertemperatur gefroren wird,
(b) optional
(b1) die gefrorene Lösung bei einer Temperatur von -25 bis -10°C getempert und
(b2) bei einer zweiten Gefriertemperatur gefroren wird, die niedriger als die Temperatur in (b1) ist, und
(c) die nach Stufe (a) oder (b) erhaltene gefrorene Lösung bei einer ersten Trocknungstemperatur von -5 bis +5°C für einen Zeitraum von mindestens 10 Stunden getrocknet wird, um eine gefriergetrocknete Zusammensetzung zu erzeugen.

2. Verfahren nach Anspruch 1, bei dem in Stufe (a) mit einer Kühlrate von 0,6 bis 1,2 °C/min auf die erste Gefriertemperatur abgekühlt wird.

3. Verfahren nach Anspruch 1 oder 2, bei dem in Stufe (a) die erste Gefriertemperatur -40°C oder niedriger, vorzugsweise -40°C bis -55°C und besonders bevorzugt -40°C bis -50°C beträgt.

4. Verfahren nach einem der Ansprüche 1 bis 3, bei dem Stufe (b) durchgeführt wird.

5. Verfahren nach Anspruch 4, bei dem in Stufe (b1) die gefrorene Lösung für einen Zeitraum von mindestens 3 Stunden, vorzugsweise mindestens 4 Stunden und besonders bevorzugt mindestens 5 Stunden getempert wird.

6. Verfahren nach Anspruch 4 oder 5, bei dem in Stufe (b1) mit einer Heizrate von 0,9 bis 1,5 °C/min auf die Temperatur für das Tempern erwärmt wird.

7. Verfahren nach einem der Ansprüche 4 bis 6, bei dem in Stufe (b2) mit einer Kühlrate von 0,9 bis 1,5 °C/min auf die zweite Gefriertemperatur abgekühlt wird.

8. Verfahren nach einem der Ansprüche 1 bis 7, bei dem in Stufe (c) die erste Trocknungstemperatur -2°C bis +2°C und vorzugsweise etwa 0°C beträgt.

9. Verfahren nach einem der Ansprüche 1 bis 8, bei dem in Stufe (c) für einen Zeitraum von mindestens 12, insbesondere mindestens 14, vorzugsweise mindestens 16 und besonders bevorzugt mindestens 18 Stunden getrocknet wird.

10. Verfahren nach einem der Ansprüche 1 bis 9, bei dem in Stufe (c) mit einer Heizrate von 0,38 bis 0,46 °C/min auf die erste Trocknungstemperatur erwärmt wird.

11. Verfahren nach einem der Ansprüche 1 bis 10, bei dem
(d) die nach Stufe (c) erhaltene gefriergetrocknete Zusammensetzung bei einer zweiten Trocknungstemperatur von +20°C bis +40°C, vorzugsweise +30°C bis +40°C und besonders bevorzugt bei etwa +40°C weiter getrocknet wird.

12. Verfahren nach Anspruch 11, bei dem in Stufe (d) mindestens 6, vorzugsweise mindestens 8 und besonders bevorzugt mindestens 12 Stunden weiter getrocknet wird.

13. Verfahren nach Anspruch 11 oder 12, bei dem in Stufe (d) mit einer Heizrate von 0,10 bis 0,25 °C/min auf die zweite Trocknungstemperatur erwärmt wird.

14. Verfahren nach einem der Ansprüche 1 bis 13, wobei die in Stufe (a) eingesetzte Lösung mindestens einen Alkohol, vorzugsweise Ethanol, Propanol oder tert.-Butanol, und besonders bevorzugt Ethanol enthält.

15. Verfahren nach einem der Ansprüche 1 bis 14, wobei die in Stufe (a) eingesetzte Lösung mindestes ein Monosaccharid, Oligosaccarid oder Zuckeralkohol, vorzugsweise Sucrose, Dextrose, Maltose, Lactose, Sorbit, Glycin, Mannit oder Dextran, und besonders bevorzugt Mannit enthält.

16. Gefriergetrocknete Zusammensetzung mit Gehalt an Bendamustin oder einem Salz davon, wobei die Zusammensetzung durch das Verfahren gemäß einem der Anspruch 1 bis 15 erhältlich ist.

## Claims

1. Process for preparing a freeze-dried composition containing bendamustine or a salt thereof, in which
(a) a solution containing bendamustine or a salt thereof is frozen at a first freezing temperature,
(b) optionally
(b1) the frozen solution is tempered at a temperature of -25 to -10°C and
(b2) is frozen at a second freezing temperature, which is lower than the temperature in (b1), and
(c) the frozen solution obtained according to step (a) or (b) is dried at a first drying temperature of -5 to +5°C for a period of at least 10 hours in order to prepare a freeze-dried composition.

2. Process according to claim 1, in which in step (a) cooling to the first freezing temperature takes place at a cooling rate of 0.6 to 1.2°C/min.

3. Process according to claim 1 or 2, in which in step (a) the first freezing temperature is -40°C or lower, preferably -40°C to -55°C and particularly preferably -40°C to -50°C.

4. Process according to any one of claims 1 to 3, in which step (b) is carried out.

5. Process according to claim 4, in which in step (b1) the frozen solution is tempered for a period of at least 3 hours, preferably at least 4 hours and particularly preferably at least 5 hours.

6. Process according to claim 4 or 5, in which in step (b1) heating to the temperature for tempering takes place at a heating rate of 0.9 to 1.5°C/min.

7. Process according to any one of claims 4 to 6, in which in step (b2) cooling to the second freezing temperature takes place at a cooling rate of 0.9 to 1.5°C/min.

8. Process according to any one of claims 1 to 7, in which in step (c) the first drying temperature is -2°C to +2°C and preferably about 0°C.

9. Process according to any one of claims 1 to 8, in which in step (c) drying is carried out for a period of at least 12, in particular at least 14, preferably at least 16 and particularly preferably at least 18 hours.

10. Process according to any one of claims 1 to 9, in which in step (c) heating to the first drying temperature takes place at a heating rate of 0.38 to 0.46°C/min.

11. Process according to any one of claims 1 to 10, in which
(d) the freeze-dried composition obtained according to step (c) is further dried at a second drying temperature of +20°C to +40°C, preferably +30°C to +40°C and particularly preferably at about +40°C.

12. Process according to claim 11, in which in step (d) further drying is carried out for at least 6, preferably at least 8 and particularly preferably at least 12 hours.

13. Process according to claim 11 or 12, in which in step (d) heating to the second drying temperature takes place at a heating rate of 0.10 to 0.25°C/min.

14. Process according to any one of claims 1 to 13, wherein the solution used in step (a) contains at least one alcohol, preferably ethanol, propanol or tert-butanol, and particularly preferably ethanol.

15. Process according to any one of claims 1 to 14, wherein the solution used in step (a) contains at least one monosaccharide, oligosaccharide or sugar alcohol, preferably sucrose, dextrose, maltose, lactose, sorbitol, glycine, mannitol or dextran, and particularly preferably mannitol.

16. Freeze-dried composition containing bendamustine or a salt thereof, wherein the composition is obtainable by the process according to any one of claims 1 to 15.

## Revendications

1. Procédé de fabrication d'une composition lyophilisée ayant une teneur en bendamustine ou un sel de celle-ci, selon lequel
(a) une solution, ayant une teneur en bendamustine ou un sel de celle-ci, est congelée à une première température de congélation,
(b) de manière facultative
(b1) la solution congelée est soumise à un traitement thermique à une température allant de -25 à -10 °C, et
(b2) est congelée à une deuxième température de congélation qui est inférieure à la température dans (b1), et
(c) la solution congelée, obtenue après l'étape (a) ou (b), est séchée à une température de séchage allant de -5 à +5 °C, pendant une durée d'au moins 10 heures, afin de produire une composition lyophilisée.

2. Procédé selon la revendication 1, selon lequel, à l'étape (a), le refroidissement jusqu'à la première température de congélation est effectué à une vitesse de refroidissement comprise entre 0,6 et 1,2 °C/min.

3. Procédé selon la revendication 1 ou 2, selon lequel, à l'étape (a), la première température de congélation est de -40 °C ou moins, de préférence de -40 °C à -55 °C, et notamment de préférence de -40 °C à -50 °C.

4. Procédé selon l'une des revendications 1 à 3, selon lequel l'étape (b) est mise en oeuvre.

5. Procédé selon la revendication 4, selon lequel, à l'étape (b1), la solution congelée est soumise à un traitement thermique pendant une durée d'au moins 3 heures, de préférence d'au moins 4 heures, et notamment de préférence d'au moins 5 heures.

6. Procédé selon la revendication 4 ou 5, selon lequel, à l'étape (b1), le chauffage jusqu'à la température du traitement thermique est effectué à une vitesse de chauffage comprise entre 0,9 et 1,5 °C/min.

7. Procédé selon l'une des revendications 4 à 6, selon lequel, à l'étape (b2), le refroidissement jusqu'à la deuxième température de congélation est effectué à une vitesse de refroidissement allant de 0,9 à 1,5 °C/min.

8. Procédé selon l'une des revendications 1 à 7, selon lequel, à l'étape (c), la première température de séchage est de -2 °C à +2 °C et est de préférence d'environ 0 °C.

9. Procédé selon l'une des revendications 1 à 8, selon lequel, à l'étape (c), le séchage est effectué pendant une durée d'au moins 12, notamment d'au moins 14, de préférence d'au moins 16 heures et notamment de préférence d'au moins 18 heures.

10. Procédé selon l'une des revendications 1 à 9, selon lequel, à l'étape (c), le chauffage jusqu'à la première température de séchage est effectué à une vitesse de chauffage allant de 0,38 à 0,46 °C/min.

11. Procédé selon l'une des revendications 1 à 10, selon lequel
(d) la composition lyophilisée, obtenue après l'étape (c), continue d'être séchée à une deuxième température de séchage allant de +20 °C à +40 °C, de préférence de +30 °C à +40 °C, et notamment de préférence d'environ +40 °C.

12. Procédé selon la revendication 11, selon lequel, à l'étape (d), le séchage est poursuivi pendant au moins 6, de préférence au moins 8 et notamment de préférence d'au moins 12 heures.

13. Procédé selon la revendication 11 ou 12, selon lequel, à l'étape (d), le chauffage jusqu'à la deuxième température de séchage est poursuivi à une vitesse de chauffage allant de 0,10 à 0,25 °C/min.

14. Procédé selon l'une des revendications 1 à 13, selon lequel la solution utilisée à l'étape (a) contient au moins un alcool, de préférence de l'éthanol, du propanol ou de l'alcool butylique tertiaire, et notamment de préférence de l'éthanol.

15. Procédé selon l'une des revendications 1 à 14, selon lequel la solution utilisée à l'étape (a) contient au moins un monosaccharide, un oligosaccharide ou de l'alcool de sucre, de préférence du saccharose, dextrose, maltose, lactose, sorbitol, glycocolle, mannitol ou dextrane, et notamment de préférence du mannite.

16. Composition lyophilisée ayant une teneur en bendamustine ou en un sel de celle-ci, ladite composition pouvant être obtenue par le procédé selon l'une des revendications 1 à 15.
